# EUROPÄISCHE PATENTANMELDUNG

(11) **EP 0 529 409 A2**
(43) Veröffentlichungstag der Anmeldung: **03.03.1993**
(21) Anmeldenummer: 92113727.9
(22) Anmeldetag: 12.08.1992
(51) Int. Cl.: G01N 15/08, G01N 33/32, G01N 27/447, B07C 5/344

(54) **Verfahren und Vorrichtung zum Prüfen der Porosität von beschichteten Gegenständen**

(30) Priorität: 22.08.1991 DE 4127740
(71) Anmelder: Jörgens, Klaus, D-42111 Wuppertal (DE)
(72) Erfinder: Jörgens, Klaus, D-42111 Wuppertal (DE)
(74) Vertreter: Rehders, Jochen, Dipl.-Ing.

(57) **Zusammenfassung**

Verfahren und Vorrichtung zum Prüfen der Porosität von beschichteten, insbesondere lackierten, elektrisch leitenden Band- und Profilmaterial oder Gegenständen, insbesondere von Hohlkörpern wie Getränkedosen, bei denen ein elektrischer Kontakt mit dem Band- und Profilmaterial oder dem Hohlkörper hergestellt wird, um einen Pol für eine angelegte Prüfspannung zu bilden, eine elektrisch leitende Verbindung zu einer beschichteten Oberfläche des Band- und Profilmaterials oder dem Hohlkörper mittels eines von einer Düse ausgehenden ununterbrochenen Strahls oder Flüssigkeitsvorhangs aus einer Elektrolytflüssigkeit hergestellt wird, um den anderen Pol für die angelegte Prüfspannung zu bilden und der von einem Pol zum anderen Pol durch den Flüssigkeitsstrahl oder Flüssigkeitsvorhang und die Beschichtung fließende Strom als Maß für die Porosität der Beschichtung gemessen wird.

## Beschreibung

Die Erfindung betrifft ein Verfahren und eine Vorrichtung zum Prüfen der Porosität von beschichteten, insbesondere lackierten, elektrisch leitenden Band- und Profilmaterial oder Gegenständen, insbesondere von Hohlkörpern wie Getränkedosen.

Die Porosität von derartigen Beschichtungen läßt sich bei Hohlkörpern dadurch messen, daß der Hohlkörper mit einer Elektrolytflüssigkeit gefüllt wird, an den Hohlkörper der eine Pol einer Prüfspannung und an eine in die Elektrolytflüssigkeit getauchte Elektrode der andere Pol der Prüfspannung angelegt und der Stromdurchgang gemessen wird. Die Prüfspannung beträgt in der Regel 6,3 V. Weist die Beschichtung keinerlei Porosität auf, wird kein Stromfluß angezeigt. Je nach Qualitätsanforderungen kann eine gewisse Porosität toleriert werden, die sich durch einen zulässigen Maximalwert des Stromflusses definieren läßt. Wird der zugelassene Schwellwert überschritten, muß der Hohlkörper markiert und/oder ausgesondert und kann ggf. einer erneuten Lackierung unterzogen werden.

Die vorbeschriebene Methode zum Prüfen der Porosität von Getränkedosen ist aufwendig, da die Getränkedosen einzeln mit Elektrolytflüssigkeit gefüllt werden müssen, anschließend die Messung durchgeführt, danach die Getränkedosen entleert, gespült und getrocknet werden müssen. Aus diesem Grunde wird das bekannte Prüfverfahren nur stichprobenartig nach dem Lackieren der Getränkedosen durchgeführt.

Das vorbeschriebene Verfahren ist nur für das Prüfen der Porosität der Innenlackierung von Getränkedosen und ähnlichen Hohlkörpern anwendbar, nicht aber für beschichtetes Band- und Profilmaterial.

Der Erfindung liegt die Aufgabe zugrunde, ein Verfahren und eine Vorrichtung zum Prüfen der Porosität von beschichteten Band- und Profilmaterial sowie von Gegenständen, insbesondere von Hohlkörpern wie Getränkedosen zu schaffen, mit denen diese Prüfung einfach und schnell durchführbar ist, so daß eine vollständige Prüfung statt einer Stichprobenprüfung erreichbar ist und das für jede Art von Beschichtung, insbesondere für elektrolackiertes Band- oder Profilmaterial oder andere lackierte Gegenstände geeignet ist.

Ausgehend von dieser Aufgabenstellung wird bei einem Verfahren der eingangs erwähnten Art vorgeschlagen, daß erfindungsgemäß ein elektrischer Kontakt mit dem Band- und Profilmaterial oder dem Hohlkörper hergestellt wird, um einen Pol für eine angelegte Prüfspannung zu bilden, daß eine weitere elektrisch leitende Verbindung zu einer beschichteten Oberfläche des Band- und Profilmaterials oder dem Hohlkörper mittels eines von einer Düse ausgehenden, ununterbrochenen Strahls oder Flüssigkeitsvorhangs aus einer Elektrolytflüssigkeit hergestellt wird, um den anderen Pol für die angelegte Prüfspannung zu bilden und daß der von einem Pol zum anderen Pol durch den Flüssigkeitsstrahl oder Flüssigkeitsvorhang und die Beschichtung fließende Strom als Maß für die Porosität der Beschichtung gemessen wird.

Ausgehend von dieser Aufgabenstellung wird des weiteren ein Verfahren zum Prüfen der Porosität von elektrolackierten Band- und Profilmaterial oder Gegenständen, insbesondere von Hohlkörpern wie Getränkedosen vorgeschlagen, bei dem erfindungsgemäß ein elektrischer Kontakt mit dem Band- und Profilmaterial oder dem Hohlkörper im Anschluß an das Elektrolackieren, aber vor dem Spülen und Trockenen hergestellt wird, um einen Pol für eine angelegte Prüfspannung zu bilden, eine elektrisch leitende Verbindung zu einer beschichteten Oberfläche des Band- und Profilmaterials oder des Hohlkörpers mittels des als Elektrolytflüssigkeit dienenden Elektrolacks oder dessen durch Ultrafiltration gewonnenes Permeat hergestellt wird, um den anderen Pol für die angelegte Prüfspannung zu bilden, wobei wenigstens ein Pol des Prüfkreislaufs elektrisch von der Anode oder Kathode der Elektrolackieranlage getrennt ist und der von einem Pol zum anderen Pol durch den Elektrolack oder das Permeat und die Beschichtung fließende Strom als Maß für die Porosität der Beschichtung gemessen wird.

Die Erfindung geht von der Überlegung aus, das Band- und Profilmaterial oder die Gegenstände in ein Elektrolytbad einzutauchen, oder die beschichtete Oberfläche des Band- oder Profilmaterials oder der Gegenstände mit einem Strahl oder einem Flüssigkeitsvorhang der Elektrolytflüssigkeit so zu beaufschlagen, daß eine ununterbrochene elektrische Verbindung zwischen der mit einem Pol der Prüfspannung kontaktierten Elektrolytflüssigkeit und dem anderen das Band- und Profilmaterial oder die Gegenstände kontaktierenden Pol und damit die Meßspannung aufrechterhalten wird. Dieser ununterbrochene Strahl oder Flüssigkeitsvorhang läßt sich so führen, daß die Elektrolytflüssigkeit die beaufschlagte Oberfläche des Band- und Profilmaterials oder der Gegenstände in einer ununterbrochenen Schicht bedeckt, so daß der Stromfluß zwischen den Polen der angelegten Prüfspannung als Maß für die Porosität der Beschichtung dienen kann.

Bei Versuchen hat sich überraschenderweise herausgestellt, daß die elektrische Verbindung über den Strahl oder den Flüssigkeitsvorhang aus ebenso wie das Eintauchen in Elektrolytflüssigkeit geeignet sind, eine ausreichend genaue Messung durchzuführen.

Wenn das Band- und Profilmaterial oder die Gegenstände nicht in ein Elektrolytbad eingetaucht werden, können sie mit großer Geschwindigkeit an den Flüssigkeitsstrahlen oder dem Flüssigkeitsvorhang vorbeigeführt werden, dennoch läßt sich die Porosität in der gewünschten Weise und für die gesamte Oberfläche des Band- und Profilmaterials und alle vorbeigeführten Gegenstände ununterbrochen durchführen. Dabei können das Band- oder Profilmaterial oder die Gegenstände taktweise oder stetig durch den Bereich des Strahls oder Flüssigkeitsvorhangs bewegt werden.

Wird die Meßspannung im eingetauchten Zustand angelegt, erfolgt die Porositätsprüfung im Anschluß und im Takt der Elektrolackierung.

Sollen alle Oberflächen des Band- oder Profilmaterials und die Außen- und Innenoberflächen von Hohlkörpern auf Porosität geprüft werden, so kann gleichzeitig oder nacheinander ein ununterbrochener Strahl oder Flüssigkeitsvorhang auf jede dieser Oberflächen gerichtet werden. Die Stromflüsse zu den unterschiedlichen Oberflächen lassen sich vorzugsweise unabhängig voneinander messen, was vorteilhaft ist, wenn die Beschichtung auf den verschiedenen Oberflächen unterschiedlich sind bzw. unterschiedliche Anforderungen an die Porosität gestellt werden.

Das erfindungsgemäße Verfahren läßt sich besonders einfach durchführen, wenn als Elektrolyt ein Lack auf Wasserbasis oder dessen durch Ultrafiltration gewonnenes Permeat verwendet wird und dieser Lack vor der Prüfung auf Porosität zum Lackieren der Oberflächen verwendet wurde. Lacke auf Wasserbasis sind stets elektrisch leitend und weisen gleichbleibende Elektrolyteigenschaften auf, so daß sich dieser Lack problemlos als Elektrolyt für das Prüfen der Porosität einsetzen läßt. Will man einen erneuten Auftrag von Pigment beim Prüfen der Porosität vermeiden, läßt sich das aus dem Lack auf Wasserbasis durch Ultrafiltration gewonnene Permeat einsetzen. Dieses Permeat entsteht immer dann, wenn sich im Lack auf Wasserbasis unerwünschte chemische Verbindungen bilden, die sich im Wasser lösen und bei einer Ultrafiltration durch den Ultrafilter hindurchtreten, während die für das Lackieren benötigten Pigmente und Bindemittel im Lack zurückbleiben.

Besonders vorteilhaft läßt sich das erfindungsgemäße Verfahren mit einem beim anodischen oder kathodischen Elektrolackieren verwendeten Lack als Elektrolyt oder dessen Permeat einsetzen.

Da beim Elektrolackieren beim Anlegen einer Gleichspannung das Phänomen der Elektrophorese, d. h. die Wanderung der geladenen Teilchen zur Anode bzw. zur Kathode stattfindet und dabei die mit Aminen neutralisierten Lackteilchen unter Filmbildung auf dem Werkstück koagulieren, bildet sich in diesem Moment eine Beschichtung, deren Porosität bereits ein Maß für die fertige, getrocknete Beschichtung darstellt. Daher ist es mit dem erfindungsgemäßen Verfahren vorteilhafterweise möglich, den Stromfluß zum Prüfen der Porosität im Anschluß an das Elektrolackieren, vor dem Spülen und Trocknen durchzuführen.

Um die Prüfspannung von der für die Elektrophorese angelegten Gleichspannung elektrisch zu trennen, kann wenigstens ein Pol des Prüfkreislaufs elektrisch von der Anode oder Kathode der Elektrolackieranlage isoliert sein.

Vorzugsweise kann der mit dem Band- oder Profilmaterial oder dem Hohlkörper durch direkten Kontakt gebildete Pol vom entsprechenden, die Anode oder Kathode bildenden Kontakt der Elektrolackieranlage isoliert sein.

In diesem Fall liegt ein Pol der Prüfspannung am Elektrolack in der Elektrolackieranlage an und ist elektrisch nicht von dem entsprechenden Pol der für die Elektrophorese benötigten Gleichspannung isoliert.

Dementsprechend lassen sich als Hohlkörper ausgebildete Gegenstände zum Elektrolackieren und zum Prüfen der Porosität mit der Öffnung nach unten auf elektrisch leitenden, elektrisch voneinander isolierten Gittern oder Rosten anordnen, während die Strahlen oder Flüssigkeitsvorhänge zum Elektrolackieren und zum Überprüfen der Porosität jeweils von unten durch die freien Zwischenräume zwischen den Gitter- bzw. Roststäben der aufeinanderfolgenden, elektrisch voneinander isolierten Bereiche der Gitter oder Roste geleitet werden.

Gemäß einer Weiterentwicklung dieses Verfahrens können die als Hohlkörper ausgebildeten Gegenstände zum Elektrolackieren mit der Öffnung nach unten auf elektrisch leitende Gitter oder Roste angeordnet sein, die Strahlen oder der Flüssigkeitsvorhang von unten durch die freien Zwischenräume zwischen den Gitter bzw. Roststäben geleitet werden, die Hohlkörper zum Prüfen der Porosität von den Gittern oder Rosten abgehoben, mit einem Pol der angelegten Prüfspannung verbunden und von unten mit den Flüssigkeitsstrahlen oder dem Flüssigkeitsvorhang als dem anderen Pol der angelegten Prüfspannung beaufschlagt werden. In diesem Fall ist es nicht nötig, aufeinander folgende, voneinander isolierte Bereiche der Gitter oder Roste vorzusehen.

Das Abheben der Hohlkörper von den Gitter-oder Rosten läßt sich vorzugsweise durch die Verwendung von Magneten zum Abheben von ferromagnetischen Hohlkörpern erreichen.

Das Band- oder Profilmaterial und die Gegenstände lassen sich bei einem Stromfluß oberhalb eines vorgebbaren Schwellenwertes markieren und/oder automatisch aussondern und können erneut bereichsweise oder ganz neu lackiert werden, indem sie in die Lackieranlage zurückgeführt oder in eine weitere, anschließende Lackieranlage geführt werden.

Ausgehend von der vorgenannten Aufgabenstellung wird des weiteren eine Vorrichtung zum Prüfen der Porosität von beschichtetem Band- und Profilmaterial oder Gegenständen vorgeschlagen, die erfindungsgemäß aus wenigstens einer Meßspannungsquelle, wenigstens einer elektischen Verbindung des einen Pols der Meßspannungsquelle mit dem Band- und Profilmaterial oder den Gegenständen, wenigstens einer elektrischen Verbindung des anderen Pols der Meßspannungsquelle mit einer Elektrolytflüssigkeit, wenigstens einer Pumpe zum Erzeugen eines gegen eine beschichtete Oberfläche des Band- oder Profilmaterials oder dem Hohlkörpern gerichteten, von wenigstens einer Düse ausgehenden ununterbrochenen Strahls oder Flüssigkeitsvorhangs und wenigstens einer Meßvorrichtung für den von einem Pol zum anderen Pol durch den Flüssigkeitsstrahl oder Flüssigkeitsvorhang und die Beschichtung fließenden Stroms als Maß für die Porosität der Beschichtung besteht.

Die Vorrichtung kann eine von der Meßvorrichtung betätigte Markier- und/oder Aussondervorrichtung aufweisen.

Die erfindungsgemäße Vorrichtung kann zum Prüfen der Porosität der Innenbeschichtung von ferromagnetischen Hohlkörpern so gestaltet sein, daß die Kohlkörper zum Elektrolackieren auf elektrisch leitenden Trag- und Führungsschienen an elektrisch entgegengesetzt gepolten Düsen gleitend vorbeitransportiert werden, nach dem Elektrolackieren in den Bereich von oberhalb der Hohlkörper angeordneten, magnetischen Trag- und Führungsschienen gelangen und von den Trag- und Führungsschienen magnetisch abgehoben werden, daß die magnetischen Trag- und Führungsschienen und die den Düsen zugeführte Elektolytflüssigkeit über Leitungen mit der Meßspannungsquelle verbunden sind und in einer der Leitungen ein Strommeßgerät angeordnet ist.

Die Erfindung wird nachstehend anhand zweier in der Zeichnung dargestellter Ausführungsbeispiele des näheren erläutert. In der Zeichnung zeigen:
Fig. 1 eine Vorrichtung zum anodischen oder kathodischen Elektrolackieren von Band- oder Profilmaterial mit anschließendem Prüfen der Porosität der Elektrolackbeschichtung und
Fig. 2 eine Vorrichtung zum anodischen oder kathodischen Elektrolackieren von Getränkedosen mit anschließendem Prüfen der Porosität der Innenbeschichtung.

Ein Verfahren und eine Vorrichtung zum anodischen oder kathodischen Elektrolackieren von Band- oder Profilmaterial sind in der deutschen Patentanmeldung P 41 16 643.4 desselben Anmelders beschrieben. Hinsichtlich der Einzelheiten des darin beschriebenen Verfahrens und der Vorrichtung wird auf diese Patentanmeldung verwiesen.

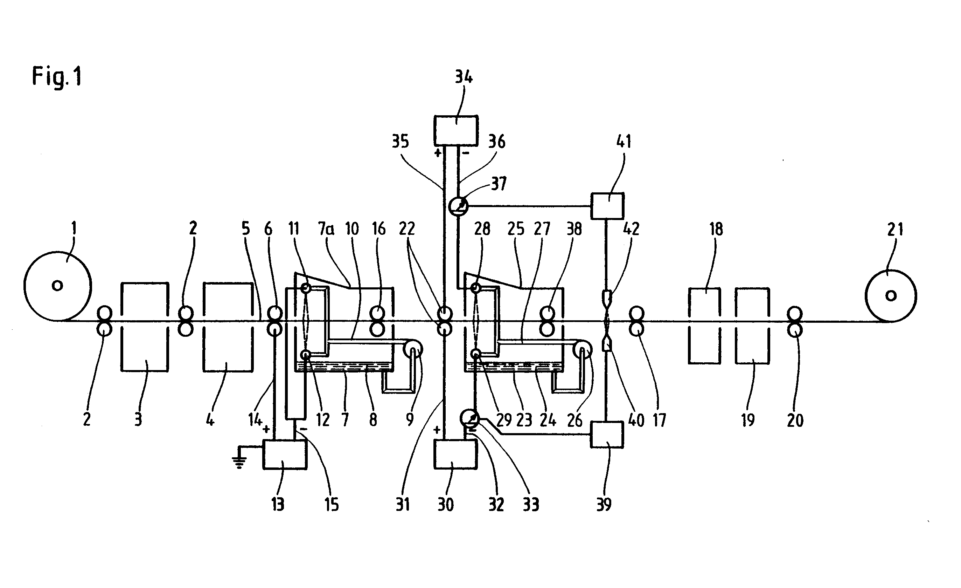

Die in Fig. 1 beschriebene Vorrichtung bezieht sich auf das Beschichten von Bandmaterial 5, läßt sich jedoch in analoger Weise für Profilmaterial einsetzen.

Während Bandmaterial 5 von einer Rolle auf einer Abhaspel 1 abgewickelt und durch die Anlage geführt wird, bis es auf einer Aufhaspel 21 wieder aufgerollt wird, kann Profilmaterial gerade durch die Anlage hindurchgeführt werden, wobei dieses Profilmaterial als Stangenmaterial in die Anlage eingeführt und aus dieser entnommen werden kann oder aber als Bandmaterial von einer auf einer Abhaspel 1 angeordneten Rolle abgewickelt, durch eine nicht dargestellte Profilieranlage hindurchgeführt und als Profil durch die Anlage hindurchgeführt werden kann. Beim Elektrolackieren von Profilmaterial entfällt die Aufhaspel 21 am Ende der Anlage und wird ersetzt durch eine nicht dargestellte Ablängvorrichtung, so daß auf Länge geschnittenes Profilmaterial gewonnen wird.

Mit Bezug auf die Beschichtung von Bandmaterial 5 stellt sich die Anlage wie folgt dar: Das von der Abhaspel 1 abgewickelte Bandmaterial 5 wird durch ein erstes Förderwalzenpaar 2, eine Reinigungsvorrichtung 3, ein weiteres Förderwalzenpaar 2, eine Spülvorrichtung 4 geführt, bis das flachliegende Bandmaterial 5 zu einem weiteren Walzenpaar 6 gelangt, das unmittelbar vor einer Beschichtungsvorrichtung angeordnet ist. Dieses Walzenpaar 6 steht über eine Leitung 14 mit dem positiven Pol einer Gleichstromquelle 13 in Verbindung und beaufschlagt auf diese Weise das Bandmaterial 5 mit dem positiven Pol der Gleichstromquelle 3, so daß das Bandmaterial 5 zur Anode wird. Der positive Pol der Gleichstromquelle 13 ist geerdet, so daß es nicht notwendig ist, die gesamte Anlage zu isolieren. Die Beschichtungsanlage besteht aus einer unterhalb des Bandmaterials 5 angeordneten Auffangwanne 7 für Elektrolytflüssigkeit 8 und einer oberhalb des Bandmaterials 5 angeordneten Abdeckhaube 7a. Eine Pumpe saugt Elektrolytflüssigkeit aus der Auffangwanne 7 an und führt sie über eine Leitung 10 einer oberhalb des Bandmaterials 5 angeordneten Düse 11 und/oder einer unterhalb des Bandmaterials angeordneten Düse 12 zu. Die Düsen 11, 12 erstrecken sich über die gesamte Breite des Bandmaterials 5 und beaufschlagen die Oberflächen des Bandmaterials 5 in einem gleichmäßigen, ununterbrochenen Strahl oder Flüssigkeitsvorhang, durch den eine elektrische Verbindung zwischen den Düsen 11, 12 und damit über einen Leiter 15 zum negativen Pol der Gleichstromquelle 13 aufrechterhalten wird. Die Düsen 11, 12 sind elektrisch isoliert von der Auffangwanne 7 und der Abdeckhaube 7a sowie der Pumpe 9 angeordnet. Während des Durchlaufs des Bandmaterials 5 zwischen den Düsen 11, 12 findet der Ladungausgleich zwischen dem als Anode wirkenden Bandmaterial 5 und den als Kathode wirkenden Düsen 11, 12 über den Elektrolytflüssigkeitsstrahl statt. Die Lackteilchen koagulieren somit auf dem Bandmaterial als Anode unter Filmbildung, wobei sich die Schichtdicke durch die Durchlaufgeschwindigkeit des Bandmaterials, den Strahlquerschnitt, die Strahlgeschwindigkeit, die durch die Gleichstromquelle 13 erzeugte Gleichspannung und/oder die Elektrolytzusammensetzung so einstellen läßt, daß eine vorgebbare, auf den beiden Oberflächen ggf. unterschiedliche Schichtdicke erreichen läßt.

Innerhalb des durch die Auffangwanne 7 und die Abdeckhaube 7a gebildeten Raumes ist ein Quetschwalzpaar 16 angeordnet, das die mitgeführte, nicht koagulierte Elektrolytflüssigkeit abquetscht.

Nach Austritt des Bandmaterials 5 aus dem Bereich der Auffangwanne 7 und der Abdeckhaube 7a, stellt ein Kontaktschleiferpaar 22 einen metallischen Kontakt zu den Oberflächen des Bandmaterials 5 her. Danach gelangt das Bandmaterial 5 in den Bereich einer mit Elektolytflüssigkeit 24 gefüllten Auffangwanne 23, die von einer Abdeckhaube 25 überdeckt ist. Mittels einer Pumpe 26 wird über eine Leitung 27 Elektrolytflüssigkeit 24 zu einer oberen Schlitzdüse 28 und zu einer unteren Schlitzdüse 29 geleitet, so daß die Oberflächen des Bandmaterials 5 mit einer ununterbrochenen Schicht von Elektrolytflüssigkeit bedeckt sind. Der Pluspol einer Meßspannungsquelle 30 ist über einen Leiter 31 mit einem Kontaktschleifer 22 verbunden, während der Minuspol über einen Leiter 32, ein Strommeßgerät 33 zur unteren Schlitzdüse 29 geführt ist. Durch die mittels der Pumpe 26 in die Düse 29 gepumpte Flüssigkeit, die in einem ununterbrochenen Flüssigkeitsvorhang gegen die Oberfläche des Bandmaterials 5 prallt, ist ein ununterbrochener elektrischer Kontakt gegeben, der sich zum Messen der Porosität der Beschichtung ausnutzen läßt. Wenn über die die Meßspannungsquelle 30 eine konstante Meßspannung von z. B. 6,3 V angelegt wird, fließt durch das Strommeßgerät 33 ein kleiner Strom, der ein Maß für die Porosität der Beschichtung ist. Überschreitet der gemessene Strom einen vorgebbaren Schwellenwert, gelangt vom Strommeßgerät 33 ein Signal zu einem Steuergerät 39, das Sprühdüsen 40 ansteuert, mit Hilfe derer eine Farbmarkierung auf dem Oberflächenbereich des Bandmaterials angebracht wird, wo eine zu große Porosität festgestellt wurde. Das Bandmaterial 5 läßt sich in diesen markierten Bereichen danach erneut lackieren.

Für die Oberseite des Bandmaterials 5 ist eine entsprechende Meßspannungsquelle 34 vorgesehen, die über einen Leiter 35 mit dem oberen Kontaktschleifer 22 und über einen Leiter 36 sowie ein Strommeßgerät 37 mit der oberen Schlitzdüse 28 verbunden ist. Die Wirkungsweise ist dieselbe und braucht im einzelnen nicht erneut beschrieben zu werden, auch nicht hinsichtlich des Steuergeräts 41 und der Sprühdüsen 42. Als Elektrolytflüssigkeit wird vorzugsweise der vorher verwendete Elektrolack oder dessen durch Ultrafiltration gewonnenes Permeat verwendet.

Danach wird das Bandmaterial 5 mittels Transportwalzen 17 einer Spülvorrichtung 18 und einer Trockenvorrichtung 19 zugeführt, von wo das Bandmaterial 5 mittels eines weiteren Transportwalzenpaars 20 abgezogen und in einer Aufhaspel 21 wieder zu einer Rolle aufgewickelt wird.

Wenn, wie bereits erwähnt, unterschiedliche Elektrolacke verwendet werden, können in nicht dargestellter Weise zwei Auffangwannen 7 mit Abdeckhaube 7a, darin angeordneten Düsen 11, 12, Quetschwalzen 16 und eine Pumpe 9 hintereinander angeordnet sein, so daß im ersten Beschichtungsschritt z. B. die Oberseite des Bandmaterials 5 mit einer Elektrolytflüssigkeit, z. B. einer bestimmten Farbe oder einer bestimmten Zusammensetzung mit einer vorgebbaren Schichtdicke beschichtet wird und danach in der folgenden Beschichtungseinheit die andere Seite des Bandmaterials 5 in gewünschter Weise beschichtet wird. Dies erlaubt es, die Ober- und Unterseite des Bandmaterials 5 verschiedenfarbig auszuführen und/oder unterschiedlichen Schichtdicken einzustellen.

In analoger Weise läßt sich in diesem Fall die Prüfvorrichtung für die Porosität der Beschichtung auf der Oberseite und der Unterseite des Bandmaterials 5 aufteilen, so daß eine Prüfvorrichtung im Anschluß an die Lackiervorrichtung für die Unterseite und eine Vorrichtung im Anschluß an die Lackiervorrichtung für die Oberseite angeordnet sein kann.

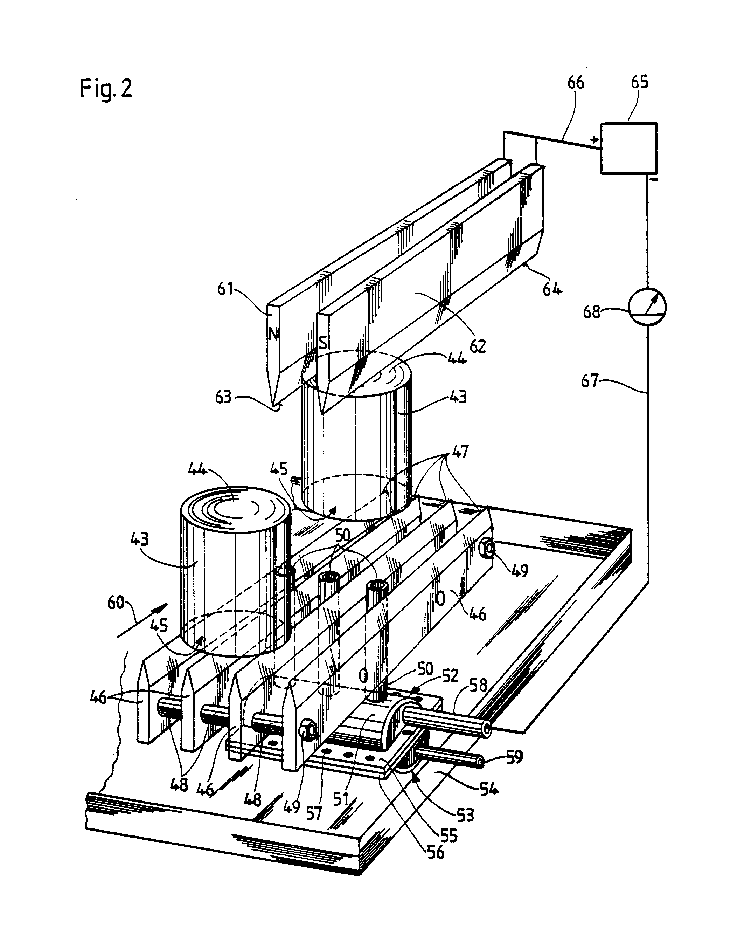

Die in Fig. 2 dargestellte Vorrichtung zum anodischen oder kathodischen Elektrolackieren von Getränkedosen ist im einzelnen in der deutschen Patentanmeldung P 40 05 620.1 vom 22. Februar 1990 desselben Anmelders beschrieben. Hinsichtlich der Einzelheiten wird auf die diesbezügliche Patentanmeldung verwiesen.

Ein metallischer Hohlkörper, insbesondere, wie dargestellt, eine Getränkedose 43, ist mit ihrem Boden 44 nach oben und der offenen Oberseite 45 nach unten auf Trag- und Führungsschienen 46 aufgesetzt.

Diese Trag- und Führungsschienen 46 sind hochkant nebeneinander und mit Abstand parallel zueinander angeordnet und werden mittels Abstandshalter 48, nicht dargestellter Gewindestifte und Muttern 49 gehalten. Die Trag- und Führungsschienen 46 bilden einen Rost für die Getränkedosen 43. Die die Getränkedosen 43 unterstützenden Schmalseiten 47 der Trag- und Führungsschienen 46 sind messerartig zugeschärft, so daß zwischen dem Dosenrand im Bereich der offenen Oberseite 45 und den Trag- und Führungsschienen 46 eine praktisch punktförmige Berührung stattfindet, die einen sehr guten elektrischen Kontakt zwischen den Trag- und Führungsschienen 46 und der Getränkedose 43 gewährleistet. Die Getränkedosen 43, von denen nur eine dargestellt ist, werden in Längsrichtung der Trag- und Führungsschienen 46 auf den messerartig zugeschärften Schmalseiten 47 entlanggeschoben, wodurch sich anlagernde Feststoffreste ständig abgeschabt werden und stets eine metallische Berührung zwischen den messerartig zugeschärften Schmalseiten 47 der Trag- und Führungsschienen 46 und dem Dosenrand gewährleistet ist.

In den Zwischenräumen zwischen den Trag-und Führungsschienen 46 sind Düsenröhren 50 angeordnet, die mit einem unterhalb der Trag- und Führungsschienen 46 angeordneten Gehäuse 51 in Verbindung stehen. Das Gehäuse 51 und die Düsenröhren 50 können aus Isoliermaterial bestehen, wenn das kathodischen Elektrolackierverfahren angewendet wird. Das Gehäuse 51 besteht aus einem einen umlaufenden Flansch 55 aufweisenden Oberteil 52 und einem einen umlaufenden Flansch 56 aufweisenden Unterteil 53, die miteinander mittels in Bohrungen 57 der Flansche 55, 56 gesteckter, nicht dargestellter Schrauben miteinander verschraubt sind.

Das Gehäuse 51 steht mit den Düsenröhren 50 und einer Elektrolytzuleitung 58 in Verbindung. Des weiteren ist eine Elektrolytrückleitung 59 vorgesehen, so daß für die nicht aus den Düsenröhren 50 austretende Elektrolytflüssigkeit eine Umwälzung im Kreislauf möglich ist.

Im Innenraum des Gehäuses 51 ist eine nicht dargestellte großflächige Elektrode angeordnet, die in elektrischer Verbindung mit dem einen Pol einer Gleichspannungsquelle steht. Die Trag- und Führungsschienen 46 sind entsprechend mit dem anderen Pol dieser nicht dargestellten Gleichspannungsquelle verbunden. Entscheidend für eine einwandfreie Beschichtung des Innenraums der Getränkedosen 43 ist, daß die aus den Düsenröhren 50 austretende Elektrolytflüssigkeit in einen ununterbrochenen Strahl bis an den Behälterboden 44 gelangt und sich von dort auf die gesamte Innenoberfläche der Dose 43 ausbreitet, so daß die Elektrophorese auf dieser Innenoberfläche ablaufen kann und eine lückenlose Beschichtung des Doseninnenraums erreicht wird.

Die Zeichnung stellt nur einen Ausschnitt einer Gesamtanlage dar, die eine große Zahl nebeneinander stehend angeordneter Hohlkörper trägt, die aneinander anliegen sowie gegenseitig und gemeinsam in Richtung des Pfeiles 60, d. h. in Längsrichtung der messerartigen Schmalseiten 47 auf diesen bewegt werden.

Statt der Schienen 46 können auch entsprechend angeordnete Drähte Anwendung finden. - Die Trag- und Führungsschienen 46 sind über einer Auffangwanne 54 für abtropfende Elektrolytflüssigkeit angeordnet.

Der über die Zuleitung 58 zugeführte Elektrolack ist über einen Leiter 67, ein Strommeßgerät 68 mit dem negativen Pol einer Meßspannungsquelle 65 verbunden, während der positive Pol dieser Meßspannungsquelle 65 über einen Leiter 66 mit parallel zueinander angeordneten magnetischen Schienen 61, 62 verbunden ist. Die eine magnetische Schiene 61 bildet einen magnetischen Nordpol, und die andere magnetische Schiene 62 bildet einen magnetischen Südpol, so daß eine in den Bereich dieser magnetischen Schienen 61, 62 gelangende Getränkedose 43 von den Trag- und Führungsschienen 46 abgehoben wird, so daß die abgehobene Getränkedose 43 nur noch mit dem Pluspol der Meßspannungsquelle 65 verbunden ist. Da der Innenraum der Getränkedose 43 weiterhin über die Düsen 50 in einem ununterbrochenen Strahl mit Elektrolytflüssigkeit, d. h. Elektrolack beaufschlagt wird, läßt sich, wie bereits bezüglich Fig. 1 beschrieben, eine Prüfung der Porosität der Innenbeschichtung der Getränkedose 43 über den Stromfluß, der im Strommeßgerät 68 angezeigt wird, durchführen.

Die Getränkedosen 43 brauchen nur wenige Millimeter von den Trag- und Führungsschienen 46 abgehoben zu werden, um eine elektrische Unterbrechung gegenüber der Stromquelle für die Elektrophorese herbeizuführen, so daß auch hier eine genaue Prüfung der Porosität möglich ist. Zwischen den Schienen 61, 62 können Dauermagnete angeordnet sein, die die angegebene Polarität erzeugen. Ebenso können auch Elektromagnete zwischen den magnetischen Schienen 61, 62 angeordnet sein, was den Vorteil bietet, daß sich die magnetische Anziehung wahlweise ein- und ausschalten läßt.

Statt der oberhalb der Getränkedosen 43 angeordneten magnetischen Schienen 61, 62 lassen sich auch Trag- und Führungsschienen 46 in Fortsetzung der dargestellten Trag- und Führungsschienen anordnen, die von den dargestellten Trag-und Führungsschienen 46 durch eine Isolierstrecke, die dem Durchmesser der Getränkedosen entspricht, elektrisch getrennt ist. Eine Düsenanordnung im Bereich der elektrisch getrennten Trag-und Führungsschienen sorgt in der bereits geschilderten Weise für einen elektrischen Kontakt zwischen den beiden Polen der Meßspannungsquelle 25, so daß sich auch in diesem Fall die Prüfung der Porosität der Innenbeschichtung schnell und einfach durchführen läßt.

Das dargestellte Beispiel bezieht sich auf das Elektrolackieren von Getränkedosen mittels Elektrolytflüssigkeitsstrahlen. Das erfindungsgemäße Verfahren läßt sich jedoch auch beim Elektrotauchlakkieren mit oder ohne Zuhilfenahme eines unter der Flüssigkeitsoberfläche gebildeten Flüssigkeitsstrahls anwenden, wenn wenigstens ein Pol der Meßspannung von einem Pol für das Elektrolackieren elektrisch isoliert ist und die Meßspannung unmittelbar im Anschluß an das Elektrolackieren, aber vor dem Spülen und Trocknen, vorzugsweise im Lackiertauchbad angelegt und der Stromfluß gemessen wird.

## Patentansprüche

1. Verfahren zum Prüfen der Porosität von beschichteten, insbesondere lackierten, elektrisch leitenden Band- und Profilmaterial oder Gegenständen, insbesondere von Hohlkörpern wie Getränkedosen mit den folgenden Verfahrensschritten:
- Herstellen eines elektrischen Kontaktes mit dem Band- und Profilmaterial oder dem Hohlkorper, um einen Pol für eine angelegte Prüfspannung zu bilden,
- Herstellen einer elektrisch leitenden Verbindung zu einer beschichteten Oberfläche des Band- und Profilmaterials oder des Hohlkörpers mittels eines von einer Düse ausgehenden, ununterbrochenen Strahls oder Flüssigkeitsvorhangs aus einer Elektrolytflüssigkeit, um den anderen Pol für die angelegte Prüfspannung zu bilden,
- Messen des von einem Pol zum anderen Pol durch den Flüssigkeitsstrahl oder Flüssigkeitsvorhang und die Beschichtung fließenden Stroms als Maß für die Porosität der Beschichtung.

2. Verfahren nach Anspruch 1, dadurch gekennzeichnet, daß der Strahl oder Flüssigkeitsvorhang eine sich über die gesamte Breite des Band- oder Profilmaterials erstreckende oder die Oberfläche des Gegenstandes bedeckende Schicht bildet.

3. Verfahren nach Anspruch 1 oder 2, dadurch gekennzeichnet, daß das Band- oder Profilmaterial oder die Gegenstände taktweise oder stetig durch den Bereich des Strahls oder Flüssigkeitsvorhangs bewegt werden.

4. Verfahren nach einem oder mehreren der Ansprüche 1 bis 3, dadurch gekennzeichnet, daß auf beide beschichteten Oberflächen des Band- oder Profilmaterials und auf die Außen-und Innenoberflächen von Hohlkörpern gleichzeitig oder nacheinander ein ununterbrochener Strahl oder Flüssigkeitsvorhang gerichtet ist und die Stromflüsse zu den unterschiedlichen Oberflächen unabhängig voneinander gemessen werden.

5. Verfahren nach einem oder mehreren der Ansprüche 1 bis 4, dadurch gekennzeichnet, daß als Elektrolyt ein Lack auf Wasserbasis oder dessen durch Ultrafiltration gewonnenes Permeat verwendet wird.

6. Verfahren nach Anspruch 5, dadurch gekennzeichnet, daß als Elektrolyt der beim anodischen oder kathodischen Elektrolackieren verwendete Lack oder dessen Permeat verwendet wird.

7. Verfahren nach Anspruch 6, dadurch gekennzeichnet, daß der Stromfluß zum Prüfen der Porosität im Anschluß an das Elektrolackieren aber vor dem Spülen und Trocknen durchgeführt wird.

8. Verfahren nach Anspruch 6 oder 7, dadurch gekennzeichnet, daß wenigstens ein Pol des Prüfkreislaufs elektrisch von der Anode oder Kathode der Elektrolackieranalge isoliert ist.

9. Verfahren nach Anspruch 8, dadurch gekennzeichnet, daß der mit dem Band- oder Profilmaterial oder dem Hohlkörper durch direkten Kontakt gebildete Pol vom entsprechenden die Anode oder Kathode bildenden Pol der Elektrolackieranlage isoliert ist.

10. Verfahren nach Anspruch 9, dadurch gekennzeichnet, daß die als Hohlkörper ausgebildeten Gegenstände zum Elektrolackieren und zum Prüfen der Porosität mit der Öffnung nach unten auf elektrisch leitenden, elektrisch voneinander isolierten Gittern oder Rosten angeordnet sind, die Strahlen oder die Flüssigkeitsvorhänge zum Elektrolackieren und zum Prüfen der Porosität jeweils von unten durch die freien Zwischenräume zwischen dem Gitter- bzw. Roststäben der aufeinanderfolgenden, elektrisch voneinander isolierten Bereiche der Gitter oder Roste geleitet werden.

11. Verfahren nach Anspruch 9, dadurch gekennzeichnet, daß die als Hohlkörper ausgebildeten Gegenstände zum Elektrolackieren mit der Öffnung nach unten auf elektrisch leitenden Gittern oder Rosten angeordnet sind, die Strahlen oder der Flüssigkeitsvorhang von unten durch die freien Zwischenräume zwischen den Gitter- bzw. Roststäben geleitet werden, die Hohlkörper zum Prüfen der Porosität von den Gittern oder Rosten abgehoben, mit einem Pol der angelegten Prüfspannung verbunden und von unten mit den Flüssigkeitsstrahlen oder dem Flüssigkeitsvorhang als dem anderen Pol der angelegten Prüfspannung beaufschlagt werden.

12. Verfahren nach Anspruch 11, gekennzeichnet durch die Verwendung von Magneten zum Abheben von ferromagnetischen Hohlkörpern von den Gittern oder Rosten.

13. Verfahren nach einem oder mehreren der Ansprüche 1 bis 12, dadurch gekennzeichnet, daß das Band- oder Profilmaterial und die Gegenstände bei einem Stromfluß oberhalb eines vorgebbaren Schwellenwerts markiert und/oder automatisch ausgesondert werden.

14. Verfahren nach Anspruch 13, dadurch gekennzeichnet, daß das markierte und/oder ausgesonderte Band- oder Profilmaterial und die markierten und/oder ausgesonderten Gegenstände erneut bereichsweise oder ganz neu lackiert werden.

15. Verfahren zum Prüfen der Porosität von elektrolackierten Band- und Profilmaterial oder Gegenständen, insbesondere von Hohlkörpern wie Getränkedosen mit folgenden Verfahrensschritten:
- Herstellen eines elektrischen Kontakts mit dem Band- und Profilmaterial oder dem Hohlkörper im Anschluß an das Elektrolackieren, aber vor dem Spülen und Trocknen, um einen Pol für eine angelegte Prüfspannung zu bilden,
- Herstellen einer elektrisch leitenden Verbindung zu einer beschichteten Oberfläche des Band- und Profilmaterials oder des Hohlkörpers mittels des als Elektrolytflüssigkeit dienenden Elektrolacks oder dessen durch Ultrafiltration gewonnenes Permeat, um den anderen Pol für die angelegte Prüfspannung zu bilden, wobei
- wenigstens ein Pol des Prüfkreislaufs elektrisch von der Anode oder Kathode der Elektrolackieranlage getrennt ist, und
- Messen des von einem Pol zum anderen Pol durch den Elektrolack oder das Permeat und die Beschichtung fließenden Stroms als Maß für die Porosität der Beschichtung.

16. Vorrichtung zum Prüfen der Prosität von beschichteten, insbesondere lackierten, elektrisch leitenden Band- und Profilmaterial oder Gegenständen, insbesondere von Hohlkörpern wie Getränkedosen mit
- wenigstens einer Meßspannungsquelle (30, 34; 65),
- wenigstens einer elektrischen Verbindung (31, 35; 66) des einen Pols (+) der Meßspannungsquelle mit dem Band- und Profilmaterial oder den Gegenständen,
- wenigstens einer elektrischen Verbindung (32, 36; 67) des anderen Pols (-) der Meßspannungsquelle mit einer Elektrolytflüssigkeit (24),
- wenigstens einer Pumpe (26) zum Erzeugen eines gegen eine beschichtete Oberfläche des Band- oder Profilmaterials oder den Hohlkörpern gerichteten, von wenigstens einer Düse (28, 29;50) ausgehenden ununterbrochenen Strahls oder Flüssigkeitsvorhangs und
- wenigstens einer Meßvorrichtung (33, 37; 68) für den von einem Pol zum anderen Pol durch den Flüssigkeitsstrahl oder Flüssigkeitsvorhang und die Beschichtung fließenden Stroms als Maß für die Porosität der Beschichtung.

17. Vorrichtung nach Anspruch 16, gekennzeichnet durch eine von der Meßvorrichtung (33, 37) betätigte Markier- und/oder Aussondervorrichtung (39, 40; 41, 42).

18. Vorrichtung nach Anspruch 16 oder 17 zum Prüfen der Porosität der Innenbeschichtung von ferromagnetischen Hohlkörpern, dadurch gekennzeichnet, daß die Hohlkörper zum Elektrolackieren auf elektrisch leitenden Trag-und Führungsschienen (46) an elektrisch entgegengesetzt gepolten Düsen (50) gleitend vorbeitransportiert weren, nach dem Elektrolakkieren in den Bereich von oberhalb der Hohlkörper angeordneten magnetischen Trag- und Führungsschienen (61, 62) gelangen, von den Trag- und Führungsschienen magnetisch abgehoben werden, daß die Trag- und Führungsschienen (61, 62) und die den Düsen (50) zugeführte Elektrolytflüssigkeit über Leitungen (66, 67) mit der Meßspannungsquelle verbunden sind und in den Leitungen (66) oder (67) ein Strommeßgerät (68) angeordnet ist.
